(19) Europäisches Patentamt
European Patent Office
Office européen des brevets

(11) EP 1 145 738 B1

(12) **EUROPEAN PATENT SPECIFICATION**

(45) Date of publication and mention
of the grant of the patent:
**12.11.2008 Bulletin 2008/46**

(51) Int Cl.:
*A61N 2/02* (2006.01)

(21) Application number: **01109187.3**

(22) Date of filing: **12.04.2001**

(54) **Magnetic stimulation device with litz wire coil**

Vorrichtung zur magnetischen Stimulation mit einer Litzenspule

Appareil de stimulation magnétique muni d'une bobine à fil de Litz

(84) Designated Contracting States:
**DE GB NL SE**

(30) Priority: **14.04.2000 JP 2000113957**

(43) Date of publication of application:
**17.10.2001 Bulletin 2001/42**

(73) Proprietor: **Nihon Kohden Corporation**
**Shinjuku-ku**
**Tokyo (JP)**

(72) Inventors:
• **Ishikawa, Norio**
**Shinjuku-ku,**
**Tokyo (JP)**

• **Suda, Shin**
**Shinjuku-ku,**
**Tokyo (JP)**

(74) Representative: **Grünecker, Kinkeldey,**
**Stockmair & Schwanhäusser**
**Anwaltssozietät**
**Leopoldstraße 4**
**80802 München (DE)**

(56) References cited:
**WO-A-99/33516**       **WO-A2-99/55421**
**GB-A- 2 111 316**       **US-A- 4 545 368**
**US-A- 5 116 304**       **US-A- 5 676 770**
**US-A- 5 984 854**

**Description**

BACKGROUND OF INVENTION

Field of invention

The present invention relates to a coil stimulator for a treatment magnetically stimulating the biological body by producing a time-varying magnetic field to induce eddy currents in the biological body by supplying pulse electric current to a litz wire coil.

Related art

As an electrical treatment apparatus for treating urinary incontinence, the present applicant has proposed a urinary incontinence treatment apparatus for magnetically stimulating biological body in JP-A-9-276418 (USP No. 5,984,854) as filed previously, in place of the well-known electrical stimulator for treating urinary incontinence by applying a stimulating electrode to the patient and supplying electrical pulses. This apparatus has a magnetic stimulating coil mounted on a coil setter installed within the room, producing a pulse-like magnetic flux in its coil repetitively to extend the magnetic flux over the range from patient's waist to lower limbs and stimulating pelvic floor muscle group and pudendal nerve with eddy currents. Also, the present applicant has proposed a magnetically stimulating coil for treatment of urinary incontinence in JP-A-10-234870, filed previously.

Besides, various kinds of coil devices for magnetic treatment have been developed in recent years.

However, the conventional coil employed a general electric wire or a copper pipe (hollow conductor). Therefore, the alternating current effective resistance of the conventional coil was so large as to cause significant electrical loss. Ultimately, the coil greatly generated heat during the use of device even by a few times, and the temperature of the coil was raised, leading to danger of burn. Thus, the conventional coil necessitated a cooling device with large capacity.

US 5,984,854 discloses a method for treating urinary incontinence which consists of delivering a train of current pulses through one or more magnetic stimulation coils to induce a train of magnetic flux pulses, which then induce an eddy current within the body, thereby to stimulate a group of pelvic floor muscles, the pudendal nerve, the external urethral sphincter, or the tibial nerve. The magnetic stimulator disclosed therein comprises an iron or ferrite core with a lite wire coil.

GB 2 111 316 A discloses a magnetic core for an electrical induction apparatus that is formed of amorphous magnetic strip material wound upon itself to form radially adjacent laminations, and it includes an inner reinforcement of non-amorphous metallic material inwardly adjacent and preferably bonded to the innermost lamination of amorphous material.

WO 99/33516 discloses a magnetic nerve stimulator system that is comprised of a core constructed from a material having a high field saturation such as oriented Si-steel, with a coil winding. A thyrister capacitive discharge circuit pulses the device. A rapidly changing magnetic field is guided by the core.

US 5,676,770 discloses the use of a non-oriented Si-steel sheets with low leakage flux for making a compact transformer.

SUMMARY OF INVENTION

It is an object of the invention to provide a practical magnetic treatment apparatus that is able to efficiently stimulate various magnetic treatment regions or diagnostic regions with a low consumption power.

To achieve an object, according to the present invention, there is provided an magnetic stimulator for stimulating nerve or muscle for treating nerve system by producing time-variable magnetic field to patient with muscle training according to claim 1.

BRIEF DESCRIPTION OF DRAWINGS

Fig. 1 is a view illustrating the cross section of a litz wire coil 6 of a device.
Fig. 2 is a cross-sectional view illustrating an example of a stimulation device using the litz wire coil 6 as shown in Fig. 1.
Fig. 3 is a graph showing eddy current density distribution arising within the biological body due to magnetic stimulation produced from the coil.
Fig. 4 is a view illustrating an example of a coil with core using the litz wire coil 6 as shown in Fig. 1.
Fig. 5 is a view illustrating another example of the coil with core using the litz wire coil 6 as shown in Fig. 1.
Fig. 6 is a view illustrating another example of the coil with core using the litz wire coil 6 as shown in Fig. 1.
Fig. 7 is a circuit diagram showing the configuration of a driving portion of a device for producing time-varying magnetic field.
Fig. 8 is a graph showing two phase waveform output from the driving portion of the device as shown in Fig. 8.
Fig. 9 is a graph showing electric field produced by the driving portion of the device as shown in Fig. 8.

DETAILED DESCRIPTION OF PREFERRED EMBODIMENTS

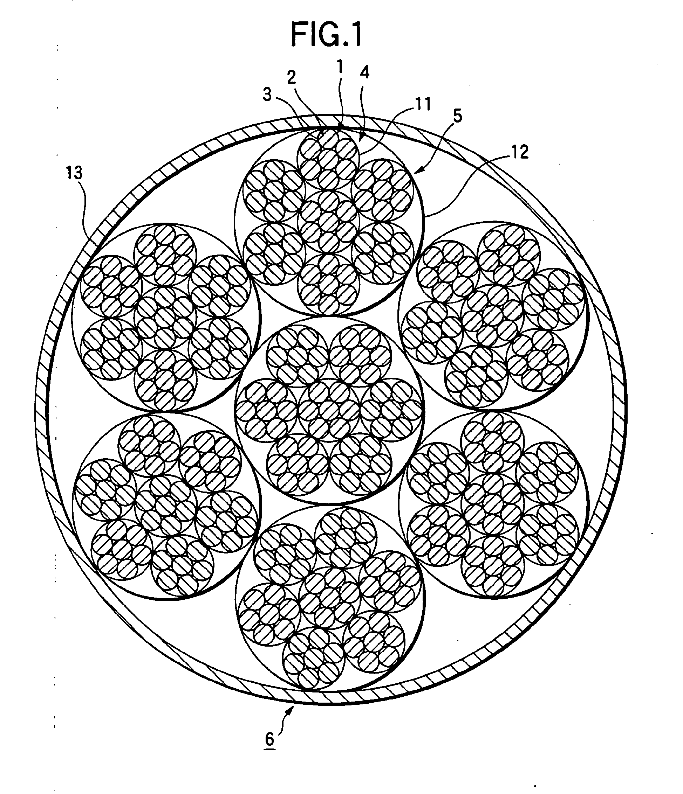

A litz wire coil for magnetic treatment of muscle or nerve system according to the present invention will be described below. Fig. 1 is a cross-sectional view of the litz wire coil 6. A bare wire 1 has a conductor portion 2 covered with an insulating layer 3. A first litz wire 4 consists of seven bare wires 1 twisted together and covered with an insulating layer 11 around its outside periphery. A second litz wire 5 consists of seven first litz wires 4 twisted together and covered with an insulating layer 12 around its outside periphery. A litz wire coil 6 consists of seven second litz wires 5 twisted together and covered with an insulating layer 13 around its outside periphery. The insulating layers 11, 12, 13 may be a glass fiber tape, a plastic tape or a rubber tape. Instead of the tapes, an insulating tube may be adopted, or a flexible adhesive of silicone may be used. The insulating layers 11 and 12 may be omitted.

Generally, conductor has a skin effect by a high frequency electric current flow, so that the electric current is concentrated on the surface to increase effective resistance and electrical loss. The skin depth $\delta$ is determined by the frequency of current in accordance with the following expression, and is reduced with larger frequency.

$$\delta = \{1/(\pi f \mu \sigma)\}^{1/2}$$

where $\mu$ is magnetic permeability, $\sigma$ is electric conductivity, and f is frequency.

The effective resistance significantly increases when the radius of conductor is $\delta$ or greater. And the diameter of conductor portion 2 for the bare wire 1 in the litz wire coil 6 and the sectional area of litz wire coil 6 are determined by the size of space for winding the coil in accordance with the above relational expression.

The bare wire 1 in the litz wire 6 shown in Fig. 1 may be made of soft copper wire, plated silver, plated thin or plated gold, Oxygen-free copper wire, or silver wire. Porous PTFE (Polyterafluoroethylene) or Blowing FEP (Fluorinated Ethylene Propylene) may preferably be applied for the insulated material of the insulated layer 3,11,12 13 in the litz wire 6. Alternatively, the insulated material may be multilayer which is constructed by winding Porous PTFE or Blowing FEP around the general insulated material such as PTFE, Porous PTFE, PFA, FEP, Blowing FEP, ETFE (Ethylene Tetrafluoroethylene), PVdF (Polyvinylidene), Milen film and Polymide film. The multilayer constructing by winding Porous PTFE ro Blowing FEP around or beneath the general jacket material such as polyethylene, nylon, vinyl chloride, or polyurethane.

When current is applied to the coil shown in Fig. 1, electromagnetic induction force is generated between the turns of the coil 6. This causes noise between the turns of the coil 6, because they are attached to one another. Therefore, in order to relieve or prevent the noise, the insulating layer 3 of the bare wire 1 in the litz wire may be within 0.025 to 2.0mm thick, the insulating layer 11 of the first litz wire 4 may be within 0.05 to 3mm thick, and the insulating layer 12 of the second litz wire 5 may be within 0.1 to 5mm thick. Moreover, the insulating layer 13 of the litz wire coil may be within 0.2 to 10 mm and may comprise acoustic insulation or noise absorbing material for reducing noise caused by contact between the turns of the coil.

The use of acoustic insulation or noise absorbing material for the particular bare wire or insulating layer may be omitted appropriately depending on the level of soundproofing effect.

Diameter of the conductor portion 2 including bare wire 1 of the litz wire coil 6 is preferably 0.2 - 1.5 mm.

The acoustic insulation or noise absorbing materials used for the insulating layers 3, 11, 12, 13 may include polyurethane foam, phenol foam, polyethylene foam, polystyrene foam, silicone, polysulfides, acrylics, denatured polysulfides, acrylic urethanes, butyl rubbers, SBRs, glass wools, rock fiber, felt, plastic sheet, and duck canvas. The property may be porous or perforated. A dry process is preferable to avoid danger of electric leakage.

A first coil driving method for treatment of nerve system employing a coil portion having the litz wire coil 6 wound has such conditions that alternate frequency is 1.0 to 3.3kHz, inductance is 10 to 50$\mu$H, pulse width is 300$\mu$s to 1ms, peak value of coil current is 2500 to 8000A, and stimulation frequency is 15 to 50Hz. This first driving method is mainly intended for the treatment with muscle training effect, and particularly favorable for treatment of stress urinary incontinence and mixed urinary incontinence as the urinary incontinence treatment.

With this first driving method, the skin effect can be reduced by the use of litz wire so that heat generated from the coil portion can be significantly decreased, even if coil current with large peak value flows.

For treatment of stress urinary incontinence and mixed urinary incontinence, a coil device is placed under the buttocks for stimulation so that magnetic flux is concentrated on pelvic floor muscle group, sacral nerve, or pudendal nerve.

A second coil driving method for treatment of nerve system employing a coil portion having the litz wire coil 6 wound has such conditions that alternate frequency is 1.0 to 3.3kHz, inductance is 10 to 50$\mu$H, pulse width is 300$\mu$s to 1ms, peak value of coil current is 1000 to 5000A, and stimulation frequency is 50 to 100Hz. This second driving method is mainly intended for the treatment with muscle training effect or treatment with thermal effect, and may be favorable for treatment of gonarthrosis, low back pain, ache, stiff neck, spasticity, carcinoma, prostatic hyperplasia, difficulty walking, bone colitio acceleration, and joint.

With this second driving method, strong contraction of muscle is more likely to arise with higher stimulation frequency, and muscle stimulation is stronger with wider pulse width in the above range of pulse width. Such stimulation with higher frequency leads to an increase in the number of current pulses per unit time flowing through the biological body, and wider pulse width increases the time for which current flows through the biological body.

Hence, current is consumed by impedance of the biological body to generate heat, obtaining thermal effect due to the generated heat. The repeated contraction and relaxation of muscle can strengthen the muscle.

The muscle training may be conducted by burst magnetic stimulation to avoid muscle fatigue due to strong contraction.

For treatment of gonarthrosis, a coil device is placed on or around patella closely and applies continuous or burst magnetic stimulation to remove or relieve pain.

For low back pain treatment, a coil device is placed around low backor waist proximately and applies continuous or burst magnetic stimulation to remove or relieve pain.

There are various causes for low back pain, which are typically classified into orthopaedic surgery region and internal medicine region, or aging and bad attitude. There is the effect of promoting blood circulation, relieving myotonia, or easing the ache by applying magnetic stimulation.

For ache treatment, a coil device is placed on aching region or effective pressure point to apply continuous or burst magnetic stimulation to relieve or alleviate the ache of the patient.

It is said that the cause of ache is algesic substance produced as a result of stimulation or damage on the aching region . There is the effect in blood flow improvement by magnetic stimulation, leading to treatment.

For treatment of stiff neck, a coil device is placed on the shoulder proximately or pressure points effective in stiff neck in shouldersand applies magnetic stimulation to alleviate the stiffness of the patient.

Spasticity is a state where stretch reflex has a low threshold and tendon reflex is accentuated so that clonus is prone to arise, and is typically caused by disturbance of pyramidal tract. Also, spasticity generally occurs with rigidity.

The effect in suppressing on a spasticity region by magnetic stimulation can be explained on the basis of the reciprocal innervation theorem. The treatment condition is such that magnetic stimulation of 80 to 100Hz is applied to spasticity muscle for 15 to 30 minutes at a magnetic stimulation intensity to cause the maximum contraction for an antagonism period of spasticity muscle. The pulse width may be selected in a range from $10\mu s$ to 1ms. Spasticity is alleviated by applying repetitive, continuous or burst magnetic stimulation to antagonist muscle of spasticity muscle.

Spasticity walking has spasticity in the limb muscle or thigh adductor owing to disturbance of pyramidal tract.

In the hyperthermia of carcinoma, the magnetic material binding agent, that is, an agent which peculiarly reacts against carcinoma tissue of the biological body that is bound with a magnetic material, is injected into the biological body, and a continuous or burst magnetic stimulation is applied to carcinoma region. Carcinoma cell is so weak to the heat as to lose proliferation potential, and die out.

It is said that carcinoma cell is typically weak to the heat, and if heated at temperatures from 42 to 45°C, particularly near 43°C, loses proliferation potential, and dies out soon. Thus, carcinoma cell is selectively heated and treated with possible least influence on the surrounding tissue.

Carcinoma can be treated by applying a time-varying magnetic field to only the carcinoma tissue and heating the tissue.

In this treatment, a conductive metal such as platinum, gold, silver or SUS is buried in the carcinoma tissue.

Next, the time-varying magnetic field is applied to the metal from outside the body. As a result, only the carcinoma tissue is heated and treatment of the carcinoma can be done.

Presently, there is an ultrasonic heating method as a non-invasive tissue heating method without heating skin plane. The ultrasonic heating method can heat the carcinoma tissue deep in the body.

But ultrasonic waves generate strong reflection or refraction in the boundary where the sound impedance is large, for example, at the boundary between air-containing parts of the body (such as the lungs) and soft tissues, or between bones and soft tissues. Therefore, the ultrasonic heating method is not suitable for the method of heating the position of the body which has plenty of gas, for example, lung, and not suitable for the method of heating the position which has bone near the position.

It is said that the causes for prostatic hyperplasia are aging and testicle. Due to these causes, hormone environment changes within the body are considered to cause prostatic hyperplasia.

For treatment of prostatic hyperplasia, a coil device is placed on prostata to apply repetitive, continuous or burst magnetic stimulation to raise the temperature of hypertrophic part.

According to the treatment, there are Hyperthermia with temperatures of 45°C or below, and thermotherapy with temperatures of 45°C to 55°C or above.

For difficulty walking treatment, a coil device is placed on waist, femoral region, leg, knee, ankle or foot to apply repetitive, continuous or burst magnetic stimulation to ease or relieve difficulty walking.

The treatment of bone colitio acceleration or joint is effective for comminuted fracture complicata, fracture owing to osteoporosis, intracervial fracture of femoral cervix, os scaphoideum fracture, infectiosis false jaw, and congenital pseudoarthrosis, and involves applying repetitive, continuous or burst magnetic stimulation to allow acceleration of bone colitio.

A third coil driving method for treatment of nerve system employing a coil portion having the litz wire coil 6 wound has such conditions that alternate frequency is 1.0 to 3.3kHz, inductance is 10 to $50\mu H$, pulse width is $300\mu s$ to 1ms, peak value of current is 1000 to 5000A, and stimulation frequency is 100Hz to 1MHz. This third driving method is mainly intended for the treatment with thermal effect, and may be favorable for blood flow improvement and bone colitio acceleration.

With this third driving method, stimulation with higher frequency leads to an increase in the number of current pulses per unit time passing through the biological body, and wider pulse width increases the time for which current flows through the biological body. Hence, current is consumed by impedance of the biological body to generate heat, giving rise to thermal effect due to generated heat.

For blood flow improvement, a coil device is placed on an insufficient blood flow region to apply repetitive, continuous or burst magnetic stimulation, to ameliorate circulation of the blood and make blood flow improvement. The factors of this thermal effect may include dermal vasodilation, blood inflow from the internal organs into the skin, acceleration of local metabolism, increased cardiac output or heart rate, fall in blood pressure, and stagnation of humor.

A fourth coil driving method for treatment of nerve system employing a coil portion having the litz wire coil 6 wound has such conditions that alternate frequency is 3.3 to 10kHz, inductance is 10 to 50$\mu$H, pulse width is 100$\mu$s to 300$\mu$s, peak value of current is 2500 to 8000A, and stimulation frequency is 1Hz to 10Hz.

This fourth driving method is mainly intended for the treatment or diagnosis with nervous stimulation, and may be particularly favorable for treatment of parkinsonian syndrome or Parkinson's disease, diagnosis of transcranial brain stimulation, treatment of epilepsy, nerve amelioration, treatment of urge urinary incontinence, and blood flow improvement. This driving method involves small pulse width, and is appropriate for nervous stimulation.

With this fourth driving method, though the alternate frequency is higher and the peak value of current is higher than in the first to third driving methods, the use of litz wire can reduce the skin effect, and decrease the heat generation in the coil portion.

For treatment of parkinsonian syndrome or Parkinson's disease, a coil device is placed proximately on the head to alleviate symptoms peculiar to parkinsonian syndrome or Parkinson's disease, or make walking movement nimble.

Parkinsonian syndrome or Parkinson's disease occurs due to disorder of nigrostriate neuron which impedes secretion of dopamin that is nerve transmitter substance in striatum.

Its main cause is considered to be disorder of dopamine biosynthesis owing to reduction of tyrosine hydroxylase.

A coil device with core for transcranial brain stimulation is provided. This coil device is placed in proximity of the head to apply repetitive, continuous or burst magnetic stimulation on a region to be stimulated within the brain without having undesirable influence on other region to be stimulated within the brain.

For treatment of epilepsy, a coil device is placed in proximity of pneumogastric nerve to apply repetitive, continuous or burst magnetic stimulation to ease or relieve epileptic fit.

For treatment of urge urinary incontinence, magnetic stimulation is applied concentrically on pelvic floor muscle group, sacral nerve, or pudendal nerve.

For blood flow improvement, a coil device is placed on an insufficient blood flow region to apply repetitive, continuous or burst magnetic stimulation so that autonomic nerve is stimulated, blood flow circulation is improved, and blood flow is improved.

As described above, the driving conditions for magnetic treatment or diagnosis are determined in accordance with various symptoms.

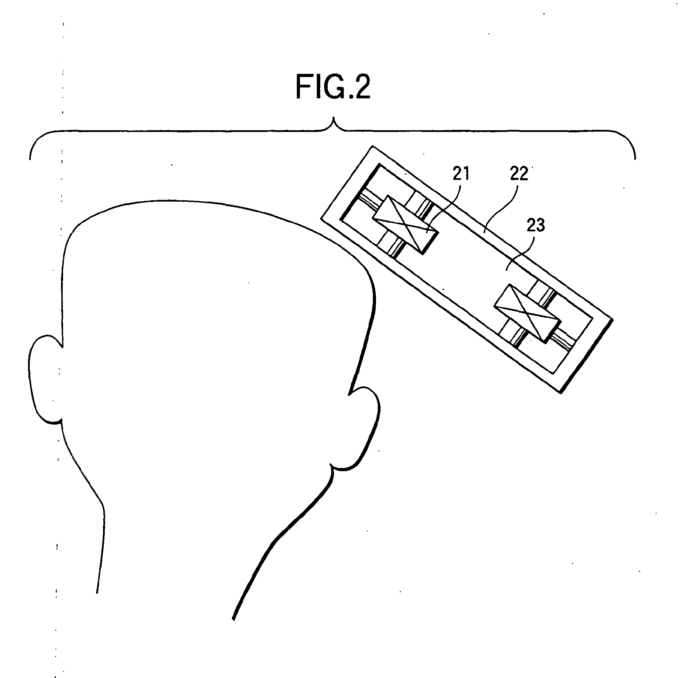

Fig. 2 illustrates a coil device for treatment of nerve system that comprises a coil portion 21 having the litz wire coil of Fig. 1 wound and a housing 22. A gap is provided between the coil portion 21 and the housing 22. The coil portion 21 uses the litz wire coil to suppress generation of heat and has the gap 23 to prevent heat produced in the coil portion 21 from passing directly to the skin of biological body to cause burning at low temperatures. Since the peak value of density of eddy current developed owing to time-varying magnetic field generated by the coil portion 21 is located closer to the coil portion than the central axis, the coil device is placed near the stimulating region. Since the effective resistance can be reduced by suppressing the skin effect, large coil current can flow. As a result, magnetic stimulation with large stimulation intensity can be applied, there is no need of providing means for obtaining large eddy current by concentrating magnetic flux employing a core. In this way, no coil may be employed because of the use of litz wire.

For treatment of urinary incontinence, the coil device having the coil portion 21 is placed under buttock to concentrate a magnetic flux on pelvic floor muscle group, sacral nerve and pudendal nerve. The turns of litz wire coil 6 may be 5 to 100.

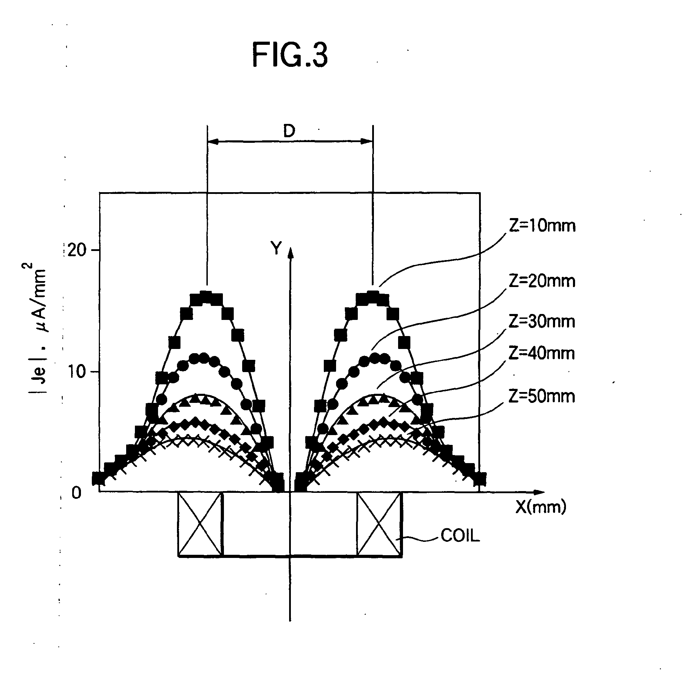

Fig. 3 is a graph showing the distribution of eddy current density for numerical analysis when the coil portion 21 is used to stimulate the biological body 21. x axis is distance from the center of coil, Y axis is absolute value of eddy current density, and Z is depth from the surface of biological body. As will be apparent from this graph, the maximum value of eddy current density distribution occurs in normal direction of coil. Accordingly, the dimension of coil may be determined in accordance with the size of stimulating region. In the case of treatment of urinary incontinence, the diameter D of coil may be about 40mm to 160mm in view of broadening in pelvic floor muscle group, sacral nerve and pudendal nerve.

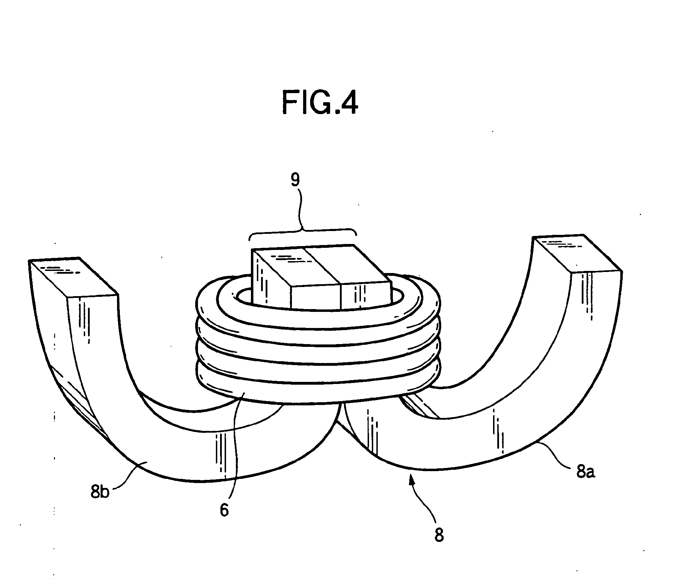

As described above, in order to enhance the stimulation efficiency by concentrating the magnetic flux, a core may be used. Fig. 4 is an appearance of the essential part of a coil device with core. As shown in Fig. 4, the core 8 is W type. The core 8 has two core elements 8a, 8b of U-character shape with one end directed upward and secured together. The core elements 8a, 8b are laminated or wound core, and may be preferably made of Permendul, grain oriented silicon steel strip, non-oriented silicon steel strip, Permalloy, Sendust, pure iron or ferrite.

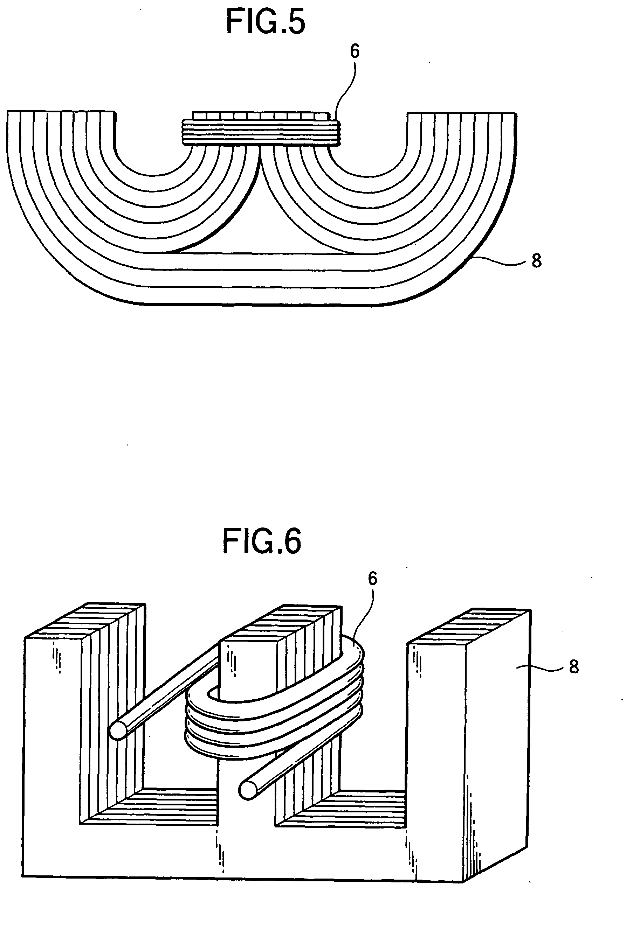

Though the core 8 is W type, it may be a cut core as shown in Fig. 5. Instead of the W type core, the laminated core 8 may be E type as shown in Fig. 6.

In particular, when the core 8 is used for treatment of urinary incontinence, the coil portion may be installed under or in the seat, and seated as a chair.

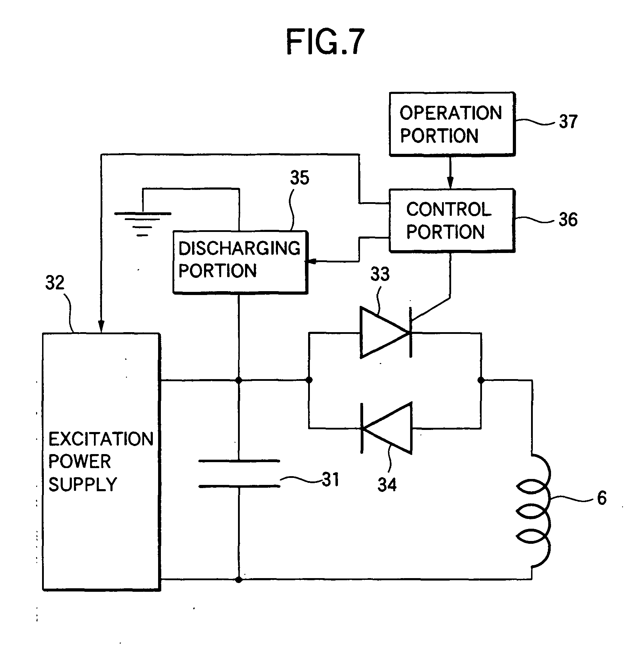

Fig. 7 shows the configuration of a driving unit for driving the litz wire coil 6. A capacitor 31 may be charged by an excitation power supply 32. The litz wire coil 6 is connected to the capacitor 31 via a circuit having a thyristor 33 and a diode 34 directed oppositely and connected in parallel. One end of the capacitor 31 is connected to a discharge portion 35. A control portion 36 controls the excitation power supply 32, the thyristor 33 and the discharge portion 35. An operation portion 37 is used to enter the data required for various instructions or controls into the control portion 36.

The operation of the coil device thus constituted will be described below. In this example, a so-called maximal stimulation intensity method will be described below in which the stimulation intensity for the patient is gradually increased. In beginning the treatment, the operator operates the operation portion 37 to gradually increase the voltage applied from the excitation power supply 32 to the capacitor 31 via the control portion 36. The control portion 36 controls such that current of one phase waveform flows through the litz wire coil 6 repetitively until the energy stored in the capacitor 31 reaches 40% of its maximal charge energy, and current of two phase waveform flows repetitively if it exceeds 40%.

Herein, if the coil device is for general purpose, the operation portion 37 may be configured to choose a treatment object, so that the control portion 36 controls appropriately the above stimulation conditions for the chosen treatment object. In the urinary incontinence treatment device, a switch for selecting the treatment object to be stress, urge, or mixed may be provided in the operation portion 37, and the control portion 36 controls the chosen treatment object under the stimulation condition.

In the case of one phase waveform, the operation of each portion is as follows. First of all, the control portion 36 controls an electric power to be supplied from the excitation power supply 32 to the capacitor 31 and the capacitor 31 to be charged. Then, the control portion 36 sends a control pulse to the thyristor 33, and turns on the thyristor 33, so that current flows from the capacitor 31 via the thyristor 33 to the litz wire coil 6. Then, the control portion 36 controls the discharge portion 35 to discharge electric energy stored in the capacitor 31. Then, the control portion 36 turns off the thyristor 33. Thereafter, the above operation is repeated.

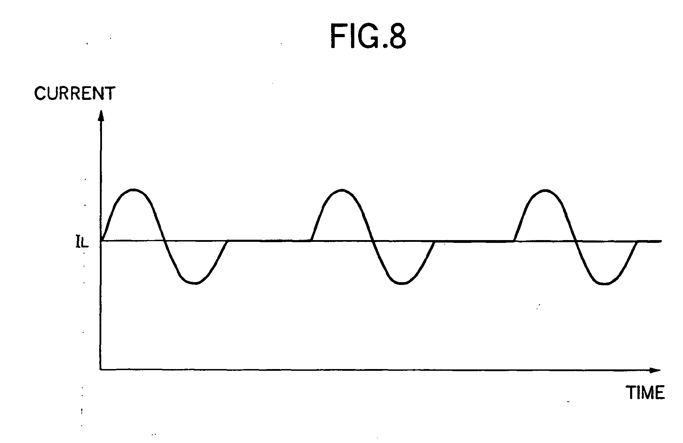

In the case of two phase waveform, the operation of each portion is as follows. First of all, the control portion 36 controls an electric power to be supplied from the excitation power supply 32 to the capacitor 31 and the capacitor 31 to be charged. Then, the control portion 36 sends a control pulse to the thyristor 33, and turns on the thyristor 33, so that current flows from the capacitor 31 via the thyristor 33 to the litz wire coil 6. And the capacitor 31 is charged again in reverse polarity. Then, the electric energy stored in the capacitor 31 is discharged to flow through the litz wire coil 6 and the diode 34. Then, the control portion 36 turns off the thyristor 33. Thereafter, the above operation is repeated. Fig. 8 shows the waveform in the coil when output in two phase waveform by driving the coil.

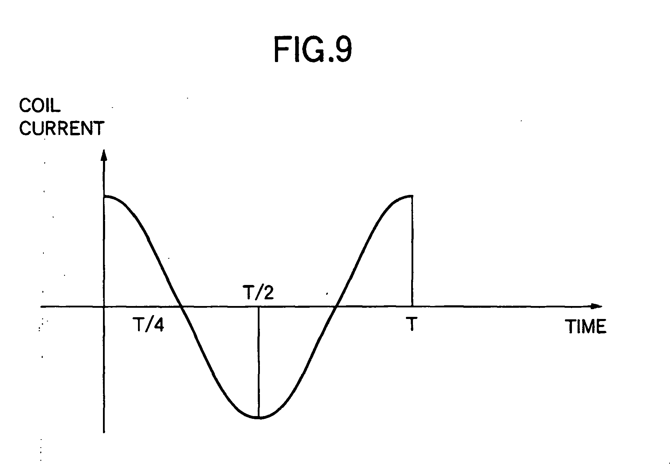

Fig. 9 shows how the electric field E varies at a certain point of the litz wire coil 6 in the case of two phase waveform. The period in the one phase waveform for which current flows through the litz wire coil 6 can be one-fourth or one-half the period T in the two phase waveform by controlling the timing of operation of the discharge portion 35 or the thyristor 33.

In this way, current flowing through the litz wire coil 6 is made in one phase waveform at first, and then in two phase waveform to attain a predetermined stimulation intensity or more. Thereby, the patient is accustomed to stimulation during the period of one phase waveform, and can be treated without giving abruptly increasing stimulation.

With this invention, a magnetic stimulator for magnetically stimulating biological body to make treatment or diagnosis by generating a time-varying magnetic field to nerve, muscle or disease part, wherein alternate frequency, inductance, pulse width, peak value of coil current, and stimulation frequency are set appropriately for the disease part, and a litz wire coil is used to produce the time-varying magnetic field. Hence, the effective alternate resistance with the skin effect can be reduced and the power consumption efficiency and stimulation efficiency can be increased. Moreover, the capacitance of capacitor can be made smaller and the device miniaturized. Since proximity effect can be suppressed, the coil diameter can be reduced while the effective resistance is not increased, and magnetic flux can be concentrated. Also, since the heat generation of coil can be decreased, burn of the patient can be avoided.

Also, with this invention, a magnetic stimulator for magnetically stimulating biological body to make treatment or diagnosis by applying a time-varying magnetic field to nerve, muscle or disease part, the litz wire coil for producing the time-varying magnetic field uses acoustic insulation or noise absorbing material for reducing noise caused by contact between wound coils parts.

**Claims**

**1.** A magnetic stimulator, comprising:

a core (8);

a litz wire coil (6) wound around the core for producing a time-varying magnetic field to stimulate a biological body, wherein said litz wire coil contains acoustic insulation or noise absorbing material for reducing noise caused by contact between coil parts; and

a power supply (32) connected to said litz wire for supplying electric variable current,

wherein

the core is comprised of non-oriented silicon steel strips; and

the core is provided as an E-core or a cut W-type core shaped as shown in Fig. 5.

2. The magnetic stimulator as set forth in claim 1, wherein the litz wire coil is wound around a center portion (9) of the core.

3. The magnetic stimulator as set forth in claim 1, further comprising a chair member, adapted to seat the biological body thereon,

wherein the litz wire coil is installed under the chair member.

4. The magnetic stimulator as set forth in claim 1, wherein the core is provided as a laminated core.

**Patentansprüche**

1. Ein magnetischer Stimulator, der umfasst:

einen Kern (8);

eine Litz-Draht-Spule (6), die um den Kern gewunden ist, zum Erzeugen eines zeitlich veränderlichen Magnetfelds, um einen biologischen Körper zu stimulieren, wobei die genannte Litz-Draht-Spule eine akustische Isolierung oder störgeräuschabsorbierendes Material zum Verringern eines Störgeräuschs, das durch einen Kontakt von Spulenpaaren verursacht wird, enthält; und

eine Energieversorgung (32), die mit dem genannten Litz-Draht zum Zuführen eines variablen elektrischen Stroms verbunden ist,

wobei

der Kern nicht-ausgerichtete Siliziumstahlstreifen umfasst; und

der Kern als ein E-Kern oder ein abgeschnittener W-Kern, der, wie es in Figur 5 gezeigt ist, geformt ist, bereitgestellt wird.

2. Der magnetische Stimulator, wie er in Anspruch 1 dargelegt ist, in dem die Litz-Draht-Spule um einen Zentralbereich (9) des Kerns gewunden ist.

3. Der magnetische Stimulator, wie er in Anspruch 1 dargelegt ist, der weiterhin ein Stuhlelement umfasst, das dazu eingerichtet ist, den biologischen Körper darauf zu setzen,

wobei die Litz-Draht-Spule unter dem Stuhlelement angebracht ist.

4. Der magnetische Stimulator, wie er in Anspruch 1 dargelegt ist, in dem der Kern als ein laminierter Kern bereitgestellt wird.

**Revendications**

1. Stimulateur magnétique, comprenant :

un noyau (8) ;

une bobine de fil divisé (6) enroulée autour du noyau pour produire un champ magnétique variable en temps pour stimuler un corps biologique, dans lequel ladite bobine de fil divisé contient un matériau d'isolation acoustique ou absorbant les bruits destiné à diminuer les bruits provoqués par le contact entre les pièces de la bobine ; et

une source d'alimentation (32) connectée au dit fil divisé destinée à amener un courant électrique variable,

dans lequel

le noyau est composé de bandes d'acier en silicium non orientées ; et

le noyau est fourni sous forme d'un noyau en E ou un noyau du type W découpé façonné comme cela est illustré sur la figure 5.

2. Stimulateur magnétique selon la revendication 1, dans lequel la bobine de fil divisé est enroulée autour d'une partie centrale (9) du noyau.

3. Stimulateur magnétique selon la revendication 1, comprenant en outre un élément de chaise, adapté pour poser le corps biologique dessus,

dans lequel la bobine de fil divisé est installée sous l'élément de chaise.

4. Stimulateur magnétique selon la revendication 1, dans lequel le noyau est fourni en tant que noyau feuilleté.

# FIG.1

# FIG.2

# FIG.3

# FIG.4

# FIG.5

6

8

# FIG.6

6

8

# FIG.7

# FIG.8

# FIG.9

COIL
CURRENT

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9276418 A
- US 5984854 A
- JP 10234870 A
- GB 2111316 A
- WO 9933516 A
- US 5676770 A